# EUROPEAN PATENT APPLICATION

(11) **EP 1 958 613 A1**
(43) Date of publication of application: **20.08.2008**
(21) Application number: 07102449.1
(22) Date of filing: 15.02.2007
(51) Int. Cl.: A61K 8/73, A61Q 19/00, A61Q 17/02, A61K 31/722, A61K 47/36, A61K 9/70

(54) **Dermal film-forming liquid formulations for drug release to skin**

(71) Applicant: POLICHEM S.A., 6912 Luxembourg (LU)
(72) Inventor: Mailland, Federico, I-20121, Milan (IT); Legora, Michela, I-22070, Appiano Gentile (CO) (IT)
(74) Representative: Pistolesi, Roberto

(57) **Abstract**

The present invention is directed to liquid compositions containing chitosan, a chitosan derivative or a physiologically acceptable salt thereof, forming a dermal film after application onto the skin, useful for delivery of actives onto the skin surface and in the *stratum corneum.*

## Description

The present invention relates to liquid compositions containing chitosan, a chitosan derivative or a physiologically acceptable salt thereof, for the preparation of a medicament, or a medical device, or a sanitary product, or a cosmetic, forming a dermal film after application onto the skin, useful for delivery of actives onto the skin surface and in the *stratum corneum.*

Delivery of active ingredients to skin, contained in drugs, sanitary products, detergents or cosmetics, often constitutes a problem in that the common formulations, like cream, ointment, gel, powder, or foam, do not allow a long lasting contact with the skin surface and often need occlusive medications to improve penetration into the skin. Occlusive medications improve the penetration of actives into the skin, but damage the superficial layers of the *stratum corneum,* leading the skin less resistant to the aggression of chemical, physical, or microbiological damages. Irritation or sensitization phenomena may occur, leading patients to stop treatment.

Chitosan derivatives are amino-polysaccharides, derived from the chitin extracted from the exoskeleton of the crustaceans, known in the art for their use in different preparations. KR20020084672 discloses chitosan as an ingredient of microspheres, useful as a carrier for separation of proteins or peptides; KR20020048534 reports chitosan as an ingredient of a pack composition for skin massage, including paraffin wax as an effective component; JP2005306746 is teaching the use of chitosan to obtain a wrinkle therapeutic agent as an ingredient of gel-like or spongy preparations of botulinus toxin. WO2005055924 reports chitosan derivatives as ingredients of hydrogels useful for cavity-filling wound dressings. JP2004231604 teaches compositions of chitosans having a high deacetylation degree, as an ingredient of a carrier sheet with a porous spongy texture. WO03042251 discloses compositions comprising chitosan in the form of a network of nano-sized fibres. WO02057983 discloses a multi-layered, air gap sheet of chitosan with a regular lamellar structure which retains drugs for a prolonged period of time; JP11060605 teaches an amphiphilic chitosan derivative which can be used as dispersion stabilizer or emulsifier in a drug for application to skin. Finally, EP1303249, discloses a nail varnish composition containing at least one antimycotic agent and hydroxypropylchitosan, whereas WO2004/112814 discloses a nail restructuring composition based on one herb extract from the genus Equisetum in combination with hydroxypropylchitosan.

The films obtained after application of nail lacquers on nail surface are generally rigid, and when accidentally applied to skin they splint easily and give a bothersome sensation on the patient's skin.

It has now surprisingly been found that liquid preparations of chitosan derivatives, besides being useful for the application to nails, may form an elastic film, after application to the skin and after evaporation of the volatile solvents, suitable to maintain drugs and other actives in intimate contact to the skin surface. The film forming solutions containing chitosans, pharmaceutically active agents and volatile solvents, may easily be sprayed onto the skin surface, by allowing quick evaporation of the volatile solvents and easy formation of an elastic film, that avoids, as an example, bothersome sensation of oily skin compared to creams, lotions and ointments, and avoids long time waiting for drying, when large skin surface is to be treated, like in *pityriasis versicolor,* an infection of the *stratum corneum* of the epidermis that requires application of antifungal agents on the whole body trunk. The film forming solutions of chitosan or chitosan derivatives may also be applied on the skin by gently massage, this is for example recommended when they are to be applied on the external genitals, or on the scalp, for the treatment of *tinea cruris,* an infection of the inguinal area, or of *tinea capitis,* a fungal infection of the scalp. The film forming solutions of chitosans allow quick penetration of actives into the deep layers of the skin, thus resulting useful for the safe treatment of skin conditions, like mycotic infections, where the fungal hyphae penetrate the *stratum corneum* and the deep layers of the skin, but also psoriasis, *lichen ruber planus* or atopic dermatitis, skin illnesses involving the keratinocytes of the skin deep layers. Moreover, the dermal film formed after evaporation of solvents of liquid chitosan compositions allows long lasting intimate contact with the skin surface, and continuous release of drugs or other actives for many hours after the application.

### DESCRIPTION OF THE INVENTION

The object of the present invention is a composition in form of a liquid formulation containing chitosan, a chitosan derivative or a physiologically acceptable salt thereof, useful for delivery of actives onto the skin surface and in the *stratum corneum.* Among chitosan derivatives, hydroxypropyl chitosan or other water soluble chitosans are preferred.

More particularly, the composition object of the present invention is represented by a medicament, or a medical device, or a sanitary product, or a cosmetic, forming a dermal film after application onto the skin.

Liquid preparations of chitosan or its derivatives, in the form of solutions, emulsions, colloids, or suspensions, with a content in chitosan or chitosan derivative from 0.1 to 10 wt.%, more preferably from 0.2 to 5 wt.%, most preferably from 0.25 to 2.0%, and containing at least one active pharmaceutical or cosmetic agent, or a plant extract, from 0.001 to 15 wt.%, more preferably from 0.2 to 10 wt.%, most preferably from 0.4 to 5.0%, are suitable to form an elastic film on the skin surface, after evaporation of solvent, said film being in intimate contact with the skin surface and allowing quick and long lasting penetration of said actives into the *stratum corneum.* The compositions obtained according to the present invention are superior to the conventional formulations, in that they can be sprayed onto the skin surface, leaving an uniform and invisible film. Moreover, the compositions according to the present invention do not dirty the dresses because they are not oily like creams or ointments are, nor leave white or coloured powder on the dresses, like powder shake products do. Furthermore, the compositions according to the present invention do not dry the skin like gels and lotions do, and do not give bothersome sensation when applied to skin, like varnishes or other rigid film forming preparations do.

Pharmaceutical compositions will be prepared according to conventional techniques, using compatible excipients and pharmaceutically acceptable carriers, and may contain, in combination, other active principles with complementary or, in any case, useful activity. Examples of these compositions prepared according to the present invention include: solutions, emulsions, suspensions, colloids, for application to nude or hairy skin, scalp, external genitals, intertriginous areas, interdigital areas.

The compositions according to the present invention contain one or more active agents from corticosteroids, immunostimulants, immununo-suppressants, antivirals, citostatics, antimycotics, antibiotics, antipsoriatic agents, keratolytics, retinoids, preservatives, insect repellents, antioxidants, plant extracts, and are suitable to treat skin illnesses, like eczema, atopic dermatitis, psoriasis, skin cancer, mycoses and other infections.

Examples of corticosteroids which may be included in the composition in accordance with the present invention include 21-acetoxypregnenolone, alclometasone or its dipropionate salt, algestone, amcinonide, beclomethasone or its dipropionate salt, betamethasone and salts thereof, including, for example, betamethasone benzoate, betamethasone dipropionate, betamethasone sodium phosphate, betamethasone sodium phosphate and acetate, and betamethasone valerate; clobetasol or its propionate salt, clocortolone pivalate, hydrocortisone and salts thereof, including, for example, hydrocortisone acetate, hydrocortisone butyrate, hydrocortisone cypionate, hydrocortisone phosphate, hydrocortisone sodium phosphate, hydrocortisone sodium succinate, hydrocortisone tebutate and hydrocortisone valerate; cortisone acetate, desonide, desoximetasone, dexamethasone and salts thereof, for example, acetate and sodium phosphate; diflorasone diacetate, fludrocortisone acetate, flunisolide, fluocinolone acetonide, fluocinonide, fluorometholone, flurandrenolide, halcinonide, medrysone, methylprednisolone and salts thereof, e.g. acetate, sodium succinate; mometasone furoate, paramethasone acetate, prednisolone and salts thereof, e.g., acetate, diethylaminoacetate, sodium phosphate, sodium succinate, tebutate, trimethylacetate; prednisone, triamcinolone and derivatives thereof, e.g. acetonide, benetonide, diacetate, hexacetonide.

Examples of immunostimulant agents which may be included in the composition in accordance with the present invention include squaric acid, imiquimod.

Examples of immunosuppressant agents which may be included in the composition in accordance with the present invention include: ciclosporin, tacrolimus, pimecrolimus and sirolimus.

Examples of antiviral agents which may be included in the composition in accordance with the present invention include acyclovir, gancyclovir, ribavirin, valacyclovir, imiquimod, idoxuridine, tromantadine, pencyclovir, edoxudine, docosanol.

Examples of citostatic agents which may be included in the composition in accordance with the present invention include fluorouracile, methotrexate, podophyllotoxin.

Examples of antimycotic agents include: 1-hydroxy-2-pyridone compounds and their salts, e.g. ciclopirox, rilopirox, piroctone, ciclopirox olamine; imidazole derivatives and their salts, e.g. clotrimazole, econazole, isoconazole, ketoconazole, miconazole, tioconazole, bifonazole, fenticonazole and oxiconazole; polyene derivatives and their salts, e.g. nystatin, natamycin and amphotericin; allylamine derivatives and their salts, e.g. naphtifine and terbinafine; triazole derivatives and their salts, e.g. fluconazole, itraconazole, terconazole and voriconazole; morpholine derivatives and their salts, e.g. amorolfine and morpholines disclosed in US-A-5,120,530; griseofulvin and related compounds, e.g. griseofulvin; undecylenic acid and its salts, in particular, the zinc and calcium salts of undecylenic acid; tolnaphtate and its salts; and flucytosine and its salts.

The antimycotic agent may also be selected from natural sources, in particular plant extracts. Examples of these extracts include tea tree oil (Melaleuca attemifolia), lavender oil (Lavandula officinalis chaix) and the leaf extract of the neem tree (Azadirachta indica).

Examples of antibiotic agents which may be included in the composition in accordance with the present invention include amoxicillin, ampicillin, benzylpenicillin, cefaclor, cefadroxil, cefatexin, chloramphenicol, ciprofloxacin, clavulanic acid, clindamycin, doxycyclin, enoxacin, flucloxacillin, kanamycin, lincomycin, minocyclin, nafcillin, nalidixic acid, neomycin, norfloxacin, ofloxacin, oxacillin, phenoxymethylpenicillin, tetracyclin and meclocycline sulfosalicylate.

Examples of antipsoriatic agents which may be included in the composition in accordance with the present invention include: anthracene derivatives, such as dithranol; psoralens, like trioxsalen or methoxsalen; Vitamin D3 analogues, like calcitriol, calcipotriol or tacalcitol; retinoids, like tazarotene, acitretine or etretinate; fumaric acid and esters thereof, e.g. monomethyl ester, dimethyl ester.

Keratolytics are peeling agents, useful to remove the horny outer layer of the skin, i.e. to promote the removal of dead skin cells from the *stratum corneum.* Examples of keratolytics which may be included in the composition in accordance with the present invention include: salicylic acid; benzoyl peroxide.

Examples of retinoids which may be included in the composition in accordance with the present invention include: retinoic acid, tretinoin, isotretinoin, etretinate and acitretin, tazarotene.

Examples of preservatives which may be included in the composition in accordance with the present invention include: benzalkonium-chlorid, benzethonium-chlorid, cetrimoniumbromid, chlorhexidin, dequaliniumchlorid, triclocarban, triclosan, salicylic acid, benzoic acid and their salts, p-hydroxybenzoic acid and its esters.

Examples of insect repellents which may be included in the composition in accordance with the present invention include: p-Menthane-3,8-diol; N,N-diethyl-meta-toluamide (DEET); 3-[N-Butyl-N-acetyl]-aminopropionic acid and its esters, in particular the ethyl ester; Pyretroids, in particular permethrin; piperidine derivatives, in particular 2-(2-hydroxyethyl)-1-piperidinecarboxylic acid and its 1-methylpropyl ester (Picaridin). The insect repellent may also be selected from plant extracts. Examples of these extracts include: oil of *Eucaliptus;* oil of Citronella *(Cymbopogon spp);* extract of *Tarchonanthus camphoratus.*

Examples of antioxidants which may be included in the composition in accordance with the present invention include: cysteine, N-acetylcisteine, glutathione.

The compositions according to the invention are applied onto the skin surface by gently massage of the concerned area, or by spray. After evaporation, an elastic film is formed onto the skin surface, that allows the continuous delivery of the actives to the skin for many hours or even for days. Moreover, the compositions according to the present invention may be sprayed on he inner surface of shoes to form e.g. an antimycotic film.

The pharmaceutical compositions and the uses of the present invention will now be more fully described by the following examples. It should, however, be noted that such examples are given by way of illustration and not of limitation.

### EXAMPLE 1

A film forming solution having the following composition wt./wt.% was prepared:

| | |
|---|---|
| 1. deionized water | 29.0% |
| 2. ethanol | 70.0% |
| 3. hydroxypropyl chitosan (HPCH) | 0.5% |
| 4. piroctone olamine | 0.5% |

### Preparation

The formulation was prepared by using a suitable closed vessel provided with a stirrer. To this vessel were added ethanol, deionized water and piroctone olamine to form a mixture. Finally, hydroxypropyl chitosan was added and the resulting mixture was stirred until dissolution.

The obtained composition had a clear and homogenous appearance even after prolonged storage. Moreover, the liquid was able to form a matte, non-sticky and elastic film which could strongly adhere to the skin surface.

### EXAMPLE 2

A controlled clinical study was performed to assess the local safety profile of the film forming solution according to the Example 1 versus a control area treated by a reference film forming solution (water 29.5%, ethanol 70.0% and HPCH 0.5%) under single blind condition.

Twenty healthy volunteers (15 females and 5 males), age range: 14 - 47 years (age mean = 26 years) were enrolled in the study. Both products were applied by the volunteers twice a day (morning and evening) for 4 weeks, by means of a roller ball, on the right and left side of the back respectively, in accordance with a previously defined randomization list. The subjects were asked to apply the product on dry skin and to take shower once a day in the morning before the application. There was no need to removal, as the film was removed by washing once a day.

No adverse event occurred during the clinical trial. When applied, the film forming solutions did not burn or cause irritation on the applied skin. The tolerability evaluation, performed by the volunteers, showed a good local safety profile of the study products.

### EXAMPLE 3

An open clinical study was performed to assess the efficacy and safety of the film forming solution according to the Example 1 on patients affected with *pityriasis versicolor,* a skin infection caused by a strain of *Malassezia furfur.*

Sixteen patients with positive microbiological test for M. *furfur* (lactophenol test) and clinical sign of hypochromia, hyperchromia, redness, and/or desquamation of the skin, itching sensation, were enrolled in the study. They applied the film forming solution by means of a spray pump twice a day by spraying all the trunk, for 21 consecutive days.

Primary objective, namely conversion to negative of microbiological test for M. *furfur,* evaluated at the end of treatment, was reached by 75% of patients. Total score of symptoms and signs (hyperchromia, redness and desquamation of the skin, itching sensation), assessed according to a four-point scale (0=absent; 1=mild; 2=moderate; 3=severe) for each sign/symptom and then added together, decreased from 4.3 at baseline to 1.2 at the end.

The tolerability was excellent in all cases and no side effects were reported.

### EXAMPLE 4

A film forming solution having the following composition wt./wt.% was prepared:

| | |
|---|---|
| 1. purified water | 13.0% |
| 2. ethanol | 73.0% |
| 3. ethyl acetate | 4.0% |
| 4. hydroxypropyl chitosan (HPCH) | 1.0% |
| 5. ciclopirox | 8.0% |
| 6. cetostearyl alcohol | 1.0% |

### Preparation

The formulation was prepared as in Example 1, by using a closed vessel with a stirrer. To this vessel were added ethanol, deionized water and ethyl acetate to form a mixture. Thereafter, cetostearyl alcohol and, after dissolution thereof, ciclopirox were added. Finally, hydroxypropyl chitosan was added and the resulting mixture was stirred for 24 hours or until dissolution.

The obtained composition had a clear and homogenous appearance even after prolonged storage. Moreover, the liquid was able to form a matte, non-sticky and elastic film which could strongly adhere to the skin surface.

### EXAMPLE 5

An *in vitro* permeation test was performed by applying the film forming solution according to the Example 4 to excised hairless mice skin, obtained from dorsal or abdominal skin of male hairless mice (strain MF1-hr-hr/Ola, Nossan srl, Correzzana, Milan, Italy) aged 7-10 weeks. Portions of the skin (ca 9 cm²), after removal of adhering fat and subcutaneous tissues, were placed as a barrier between the two compartment of Gummer permeation vertical cells (Gummer, C.L. et al. The skin penetration cell: design update. Int. J. Pharm. 1987, 40, 101-104). The receiving phase was introduced into the lower compartment and 75.0 µL of the composition according to the Example 4 were regularly distributed on the exposed skin surface. At predetermined time intervals (1, 2, 3, 4 and 5 hours) 5.0 mL of the receiving solution were collected for analysis and immediately replaced by an equal volume of fresh buffer. The experiment was replicated 6 times.

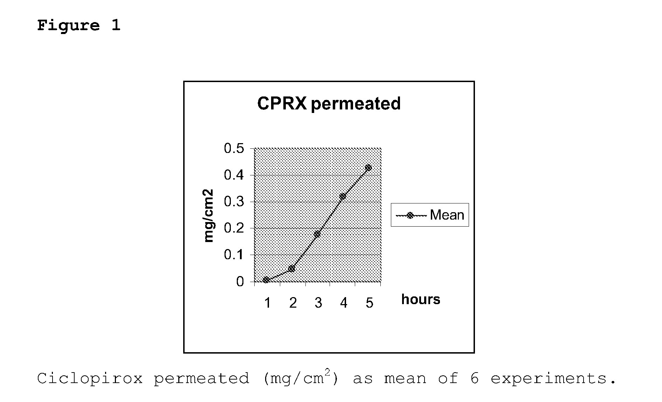

The ciclopirox permeated through hairless mice skin in the 6 experiments is reported in Table 1 and Figure 1.

**Table 1: ciclopirox permeated (mg/cm²) in the 6 individual experiments**

| | test 1 | test 2 | test 3 | test 4 | test 5 | test 6 |
|---|---|---|---|---|---|---|
| h 1 | 0.001548 | 0.002964 | 0.001542 | 0.001617 | 0.00487 | 0.020379 |
| h 2 | 0.011342 | 0.050657 | 0.012408 | 0.044503 | 0.046957 | 0.118199 |
| h 3 | 0.056009 | 0.101967 | 0.049021 | 0.174453 | 0.272469 | 0.408437 |
| h 4 | 0.114553 | 0.122609 | 0.08552 | 0.275656 | 0.527261 | 0.762349 |
| h 5 | 0.172149 | 0.14013 | 0.151676 | 0.327962 | 0.721243 | 1.025413 |

It is concluded that ciclopirox was able to permeate the mice skin, after the application of the film forming solution of hydroxypropyl chitosan according to the Example 4, in a quick and long lasting way.

### EXAMPLE 6

A film forming solution having the following composition wt./wt.% was prepared:

| | |
|---|---|
| 1. purified water | 28.0% |
| 2. ethanol 95% | 70.0% |
| 3. hydroxypropyl chitosan (HPCH) | 0.5% |
| 4. piroctone olamine | 0.5% |
| 5. climbazole | 0.5% |
| 6. Croduret 40DL® (1) | 0.5% |

| | |
|---|---|
| (1) PEG-40 hydrogenated castor oil | |

### Preparation

The formulation was prepared as in Examples 1 and 4, by adding hydroxypropyl chitosan as the final ingredient, and stirring the mixture for 24 hours or until dissolution.

The obtained composition had a clear and homogenous appearance even after prolonged storage. Moreover, the liquid was able to form a matte, non-sticky and elastic film which could strongly adhere to the skin surface.

### EXAMPLE 7

A film forming liquid suspension having the following composition wt./wt.% was prepared:

| | |
|---|---|
| 1. purified water | 24.5% |
| 2. ethanol | 68.0% |
| 3. hydroxypropyl chitosan (HPCH) | 0.5% |
| 4. tolnaftate | 1.0% |
| 5. Transcutol P® (1) | 6.0% |

| | |
|---|---|
| (1) diethylenglycole monoethyletere | |

### Preparation

The formulation was prepared by using a suitable closed vessel provided with a stirrer. To this vessel were added

Transcutol P and tolnaftate. The mixture was stirred and to the final suspension ethanol and water were added. After short stirring hydroxypropyl chitosan was added. The misture was stirred for 2 hours until complete dispersion of hydroxypopyl chitosan. The resulting composition is a fine suspension of tolnaftate that tends to decant, but is easily resuspended after short shake.

### EXAMPLE 8

An acceptability test was performed by spraying the film forming composition according to the Example 7 to the dorsal area of the left foot of a subject. As a comparison, a reference product from the market (Lamisil® AF Defense Tolnaftate 1% Antifungal Shake Powder), having the following composition according to the manufacturer: tolnaftate 1% wt/wt, corn starch, fragrance, talc, was applied on the same areas of the right foot. Both suspensions had to be shake before use.

When sprayed onto the skin of the left foot, the composition according to the Example 7 evaporated in 1-2 minutes, by forming an invisible and elastic film which could strongly adhere to the skin surface. On the contrary, when sprayed onto the skin of the right foot, the reference (Lamisil®) product evaporated in about 2-3 minutes, by leaving a white layer of powder that cracked all over soon after drying.

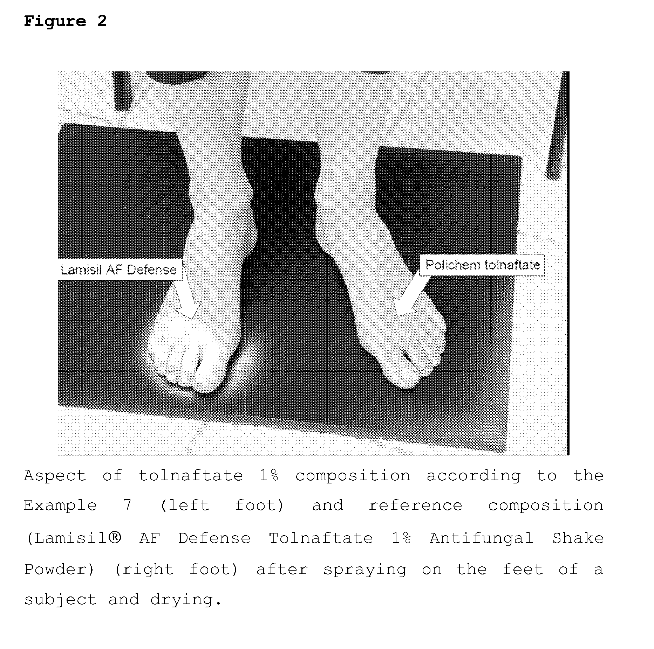

The aspect of the two products after spraying and drying is reported in the Figure 2.

### EXAMPLE 9

A film forming solution having the following composition wt./wt.% was prepared:

| | |
|---|---|
| 1. deionized water | 23.7% |
| 2. ethanol 95 | 70.0% |
| 3. hydroxypropyl chitosan (HPCH) | 0.3% |
| *4. Melaleuca alternifolia* extract | 5.0% |
| 5. Hydrogenated castor oil | 1.0% |

### Preparation

The formulation was prepared as in Examples 1 and 4, by adding hydroxypropyl chitosan as the final ingredient, and stirring the mixture for 24 hours or until dissolution.

The obtained composition had a clear and homogenous appearance even after prolonged storage. Moreover, the liquid was able to form a matte, non-sticky and elastic film which could strongly adhere to the skin surface.

### EXAMPLE 10

A preparation having the following composition wt./wt.% was prepared:

| | |
|---|---|
| 1. purified water | 19.45% |
| 2. propylene glycol | 10.00% |
| 2. isopropanol | 70.00% |
| 3. chitosan | 0.50% |
| 4. bechlometasone dipropionate | 0.05% |

### Preparation

The formulation was prepared by dissolving chitosan and bechlometasone dipropionate in propylene glycol, then adding the other ingredients, and stirring the mixture until dissolution. The resulting liquid was able to form an elastic film which could strongly adhere to the skin surface.

### EXAMPLE 11

A preparation having the following composition wt./wt.% was prepared:

| | |
|---|---|
| 1. purified water | 33.6% |
| 2. ethanol | 65.0% |
| 3. hydroxypropyl chitosan | 0.5% |
| 4. *Tarchonanthus camphoratus* essential oil | 0.3% |
| 5. *Melaleuca alternifolia* essential oil | 0.5% |
| 6. Menthyle lactate | 0.1% |

### Preparation

To the ethanol under stirring menthyle lactate, *M.alternifolia* essential oil and *T.camphoratus* essential oil were added. After complete dissolution, water was gradually added; to the limpid mixture under stirring hydroxypropyl chitosan was added; after complete dispersion of the polymer, a solution was obtained, which was limpid, colorless, with a characteristic alcoholic-aromatic odor.

### EXAMPLE 12

An open clinical study was performed to assess the insect repellent efficacy and the safety profile of the film forming solution according to the Example 11 on humans exposed to the bites of mosquitoes during summer. Efficacy endpoints of the study were insect repellent effect, lenitive effect after insect bite and cosmetic acceptability. The test was performed on 21 subjects, 19 females and 2 males, aged between 21 and 65 years, that gave their informed consent.

All subjects performed a topical treatment, at least once-a-day for 10 days, with the test product according to the Example 11. The efficacy as insect repellent was positively judged by 48% of treated subjects. Lenitive efficacy after bite was positively judged by 80% of subjects, namely 40% a marked/very marked decrease, 30% a mean decrease and 10% a slight decrease.

Cosmetic acceptability was generally judged as good. During the study, no adverse events occurred, and tolerability of the product according to the Example 11 was judged as optimal by 62% and good by 38% of patients.

## Claims

1. Use of:
(a) a chitosan, a chitosan derivative or a physiologically acceptable salt thereof,
(b) at least a pharmaceutical or a cosmetic active ingredient, and
(c) at least one volatile solvent,
for the manufacture of a liquid composition forming a dermal film after application onto the skin subsequently to the evaporation of volatile solvent (c).

2. Use according to claim 1, wherein said composition delivers active principles onto the skin surface.

3. Use according to claim 1, wherein component a) is present in an amount of 0.1 to 10 wt.%, with respect to the total weight of the composition.

4. Use according to claim 2, wherein component a) is present in an amount of from 0.2 to 5 wt.%, preferably from 0.25 to 2.0%, with respect to the total weight of the composition.

5. Use according to claims 1-4, wherein component a) is a water soluble chitosan derivative or a salt thereof.

6. Use according to claims 1-5, wherein said water soluble chitosan derivative is hydroxypropylchitosan.

7. Use according to any of the preceding claims, wherein component b) is selected from corticosteroids, immunostimulants, immununo-suppressants, antivirals, citostatics, antimycotics, antibiotics, antipsoriatic agents, keratolytics, retinoids, preservatives, insect repellents, antioxidants, plant extracts, or combinations thereof.

8. Use according to claim 7, wherein component b) is one or more from the group including bechlometasone dipropionate, clobetasol, piroctone and its salts, ciclopirox and its salts, terbinafine and its salts, climbazole, tolnaftate, clotrimazole, cyclosporin, imiquimod, acyclovir, calcipotriol, tazarotene, salicylic acid, plant extract from *Tarconanthus camphoratus* and/or from *Melaleuca alternifolia.*

9. Use according to any of the preceding claims, wherein component b) is present in an amount of from 0.001 to 15 wt.%, more preferably from 0.2 to 10 wt.%, most preferably from 0.4 to 5.0% , with respect to the total weight of the composition.

10. Use according to any of the preceding claims, wherein the component c) is a lower alkanol.

11. Use according to the claim 10, wherein the lower alkanol is ethanol or isopropanol.

12. Use according to claims 10 and 11, wherein water is the solvent or a co-solvent.

13. Use according to any of the preceding claims, wherein said liquid formulation includes customary excipients and/or adjuvants.

14. Use according to any of the preceding claims, wherein said liquid formulation is suitable to treat skin conditions and illnesses, like dandruff, eczema, atopic dermatitis, psoriasis, mycoses, *pityriasis versicolor* and other infections.

15. Use according to any of the preceding claims, wherein said liquid formulation is in a form suitable for application to nude or hairy skin, scalp, external genitals, intertriginous areas, interdigital areas.

16. Use according to any of the preceding claims, wherein said liquid formulation includes solutions, emulsions or suspensions.

17. Use according to any of the preceding claims, wherein said liquid formulation is applied by spray.
